# EUROPEAN PATENT SPECIFICATION

(11) **EP 4 260 900 B1**
(45) Date of publication and mention of the grant of the patent: **06.05.2026**
(21) Application number: 20964801.3
(22) Date of filing: 14.12.2020
(51) Int. Cl.: A61N 1/36, A61N 1/05, A61B 6/12, A61B 90/00

(54) **SLICED ELECTRODE AND IDENTIFICATION METHOD THEREFOR**
IN SCHEIBEN GESCHNITTENE ELEKTRODE UND IDENTIFIZIERUNGSVERFAHREN DAFÜR
ÉLECTRODE DÉCOUPÉE ET PROCÉDÉ D'IDENTIFICATION CORRESPONDANT

(30) Priority: 11.12.2020 CN 202011444194
(43) Date of publication of application: 18.10.2023
(73) Proprietor: SceneRay Co., Ltd., Jiangsu 215123 (CN)
(72) Inventor: YI, Che, Suzhou, Jiangsu 215000 (CN); ZHU, Yongwei, Suzhou, Jiangsu 215000 (CN); ZHANG, Haitao, Suzhou, Jiangsu 215000 (CN)
(74) Representative: Cabinet Beaumont
(86) International application number: PCT/CN2020/136052
(87) International publication number: WO 2022/120868

(56) References cited:
- CN-A- 105 263 567
- CN-A- 106 693 163
- CN-A- 108 992 774
- US-A1- 2008 269 854
- US-A1- 2020 230 397
- US-B2- 10 067 659
- US-B2- 8 560 085

## Description

### TECHNICAL FIELD

The present invention relates to the field of medical technologies, and in particular, to a directional lead and an identification method therefor.

### BACKGROUND

Every year, thousands of patients undergo the deep brain stimulation (DBS) surgery for treating Parkinson, obsessive-compulsive disorder, autism or the like, and matched medical appliances such as corresponding stimulators and electrode leads are implanted into the bodies of the patients. Traditional electrode leads employ annular stimulation contacts, and the annular stimulation contacts spread the electrical stimulation all around to achieve the treatment effect. However, the excessive treatment may cause some undesirable effects on brain sites not associated with treatment.

In recent years, with the development of medical research, people begin to recognize that the stimulation point direction and the stimulation pulse after the electrode is implanted into the brain have significant influence on the treatment effect, and the requirement of providing different directions and different electrical stimulation pulses in a same ring-shaped circumferential direction arises. In order to improve the treatment precision, researchers divide an annular electrode into pieces, namely, multiple segments and multiple columns of electrode slices are arranged on one cylindrical annular surface, so that the generated electric stimulation can be directed at specific sites, thereby reducing the excessive treatment. However, for this directional lead, in the current actual use process, the medical staff cannot quickly and accurately position a certain one or several electrode slices so as to achieve the fixed-point electrical stimulation. Therefore, there is an urgent need for doctors to develop a mark identification method with low cost and high efficiency.

Further relevant technologies are also known from US2020230397A1 which relates to imaging markers for stimulator leads, US8560085B2 which relates to methods for making leads with segmented electrodes for electrical stimulation systems and US 10067659B2 which relates to systems and methods for determining orientation of an implanted lead.

### SUMMARY

The invention is set out in the appended set of claims.

### BRIEF DESCRIPTION OF DRAWINGS

FIG. 1 is a schematic structural diagram of a directional lead with a marking pattern according to the present invention;
FIG. 2 shows an imaging of a directional lead without the marking pattern;
FIG. 3 is a schematic structural diagram of different marking patterns, where FIG. 3(a) is an isosceles triangular pattern, FIG. 3(c) is a trapezoid pattern, FIG. 3(d) is a zigzag pattern, and FIG. 3(b) is a non-isosceles triangular pattern; and
FIG. 4 shows imagings of a directional lead in different poses when a marking pattern is an isosceles triangular pattern.

### Reference numeral list

- 1: connection portion
- 2: electrode slice
- 3: marking pattern

### DETAILED DESCRIPTION

The present invention is further described below in conjunction with the drawings and the specific embodiments so that the present invention can be better understood by those skilled in the art and can be implemented, but the listed embodiments are not to be construed as limiting the present invention.

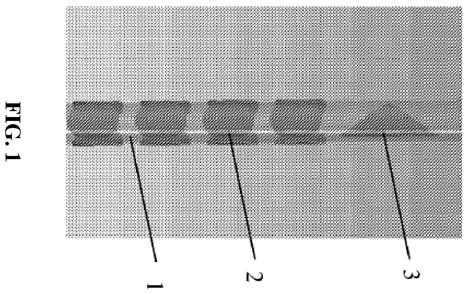

As shown in FIG. 1, the present invention discloses a directional lead. The directional lead includes a connection portion 1, and multiple electrode slices 2 which circumferentially surround the connection portion and are arranged in an array. The connection portion has a columnar structure, and a marking pattern 3 is disposed on a side surface of the connection portion 1. The marking pattern 3 is located on a side of the electrode slices 2 and at least includes three distinguishing feature portions. The distinguishing feature portions are sharp corners or rounded corners, and positions of the electrode slices can be positioned by the distinguishing feature portions of the marking pattern 3. The working principle of the present invention is that, since the marking pattern 3 has three distinguishing feature portions, a transmission image of the directional lead is acquired when the directional lead is rotated, and the three distinguishing feature portions can form different combinations of shapes in different transmission images, so that the position of a certain electrode or several electrodes on the directional lead at this time can be distinguished.

In the present invention, the electrode slice 2 is disposed on an outer periphery of the connection portion, a conductive wire is located inside the connection portion, or is molded on a same surface with the electrode slice by using the same process molding, or is located on a back surface of the electrode slice, so that the conductive wire is connected to the electrode slice 2 on the side surface of the connection portion 1 for supplying the power.

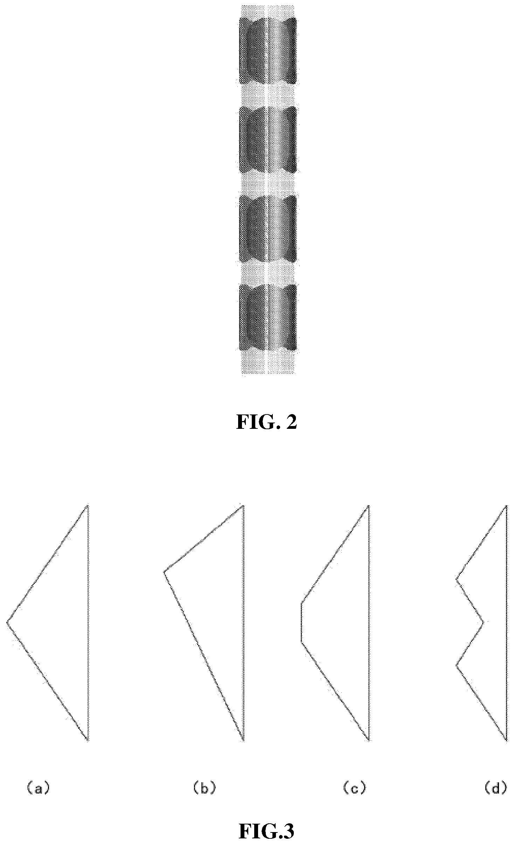

As shown in FIG. 2, in the medical imaging of an existing directional lead, due to the significant difference in material between the electrode slices and the surrounding area, the electrode slices can be clearly seen, but the material in the surrounding area are penetrated and cannot be displayed, and therefore, the imaging may be regarded as a superposition of a front view and a back view, which undoubtedly increases the difficulty in identifying an actual condition of an electrode contact on a two-dimensional picture. In an actual overlapped image, if identification is to be performed by a two-dimensional medical image (X-ray), it is impossible to distinguish whether a graphic shape (which is symmetrical along a central axis), such as a circle, a rectangle or a square, is located on the front side or on the back side after image overlapping. For a symmetrical pattern different from the above-described symmetrical pattern, in consideration of the superimposition of the front view and the back view at a specific angle, only when the image is photographed at a specific angle, it can be ensured that the doctors can identify the orientation of the electrode, which requires a specific limitation on a photographed angle. Therefore, in an existing solution, photographing for medical images at multiple angles is required to help the doctors and the researchers identify the serial number of the directional lead.

In the present invention, the marking pattern is provided and a size occupied by the marking pattern itself on the side surface of the connection portion is small, so that more positions can be left to facilitate the wire routing and other structural designs. By means of simple teaching, the doctors and the researchers can determine a direction through a two-dimensional image in multiple planes, thereby reducing the orientation time required in the surgical process and the subsequent visit, and reducing the unnecessary radiation to patients.

As shown in FIGS. 1 and 3, the marking pattern includes a triangular pattern, that is, the marking pattern described above has three distinguishing feature portions, and the distinguishing feature portions are sharp corners. The triangular pattern is an asymmetric graphic with respect to the first line, where the first line is a line that is parallel to a central axis of the connection portion and located on the side surface of the connection portion. As shown in FIG. 3, the marking pattern is composed of multiple triangular patterns, and the marking pattern is an asymmetric image with respect to the first line. The marking pattern may be various patterns, in which FIG. 3 (a) is an isosceles triangular pattern, FIG. 3 (c) is a trapezoid pattern, which may be composed of two triangular patterns, FIG. 3 (d) is a zigzag pattern, which may be composed of multiple triangular patterns, and FIG. 3 (b) is a non-isosceles triangular pattern. For the design of the marking pattern, it is only necessary to ensure that the images of the marking pattern are different when the directional lead is rotated to different states. The marking pattern may be located on a non-electrode slice region, but not limited to above the electrode, or the electrode slice itself may be marked by using the same principle. The marking pattern may be the same as, but not limited to, the material and the process of the electrode slice, and may be also an additional part or process molding.

Hereinafter, the present invention will be explained and described in detail with respect to the triangular pattern.

After the triangular pattern is symmetrically mirrored (when the triangle is turned to a back surface of a cylinder, a mirrored picture is generated), the doctors and the researchers can still be assisted in the graphic recognition due to different orientations of vertexes. There are also many choices for a triangular pattern, such as an isosceles (equilateral) triangle and a right-angled triangle. In a preferred solution, the symmetrical mirror pattern of the isosceles triangle has more features, and has advantages over the right triangle. A bottom edge of the isosceles triangular pattern is parallel to the central axis of the connection portion.

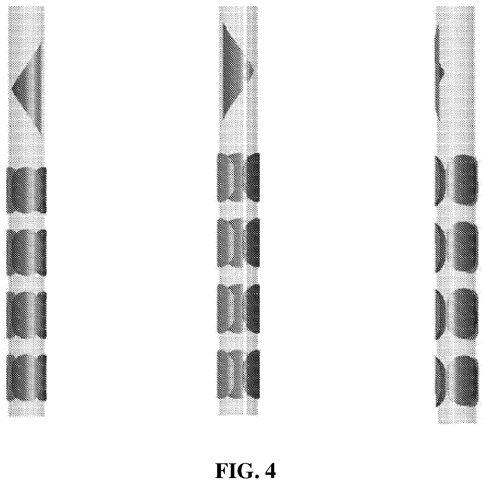

FIG. 4 shows imagings of the directional lead in different poses when the marking pattern is an isosceles triangular pattern. As can be seen from the drawing, the imagings of the isosceles triangular pattern are different when the directional lead is in different states, and therefore, it is convenient for the medical personnel to quickly know the position of the electrode slice. In this manner, the electrode slice is positioned.

Two ends of the marking pattern and a center point of a radial cross-section of the connection portion form an angle of 105° - 135° on the radial cross-section of the connection portion. Specifically, when the marking pattern is the isosceles triangular pattern, a triangular circumferential dimension is designed to be 1 mm, an area of the overall marking pattern should be not less than a dimension of a single electrode slice, i.e., 1.40mm², a triangular axial dimension is set to be 3 mm, and the triangular pattern occupies only one third of the entire circumference, that is, 120°.

In another embodiment, the present invention further discloses an identification method for a directional lead, and the identification method includes steps described below.

In step one, a marking pattern is provided in a non-electrode slice region of the directional lead. When the directional lead is rotated, imaging positions or shapes of the marking pattern are different. The marking pattern includes a triangular pattern, the triangular pattern is an asymmetric graphic with respect to the first line, and the first line is a line that is parallel to a central axis of the directional lead and is located on the side surface of the directional lead. In order to facilitate positioning of the electrode slices, the directional lead includes a connection portion and multiple electrode slices circumferentially surrounding the connection portion and arranged in an array. For the directional lead according to the present invention, it may be implemented by combining the annular electrode and the electrode slice, that is, for such electrode, a partial region is provided with an annular electrode, and a partial region is provided with multiple electrode slices. The marking of the marking pattern may also be designed and selected with reference to the solution of the previous embodiment.

In step two, an image of the directional lead is acquired by a transmission optical imaging device. The transmission spectral imaging device performs an X-ray scan or an electronic computer tomography scan. A two-dimensional transmission image of the directional lead may be acquired and obtained through the transmission optical imaging device.

In step three, a position of the electrode slice is positioned through an imaging of the marking pattern on the image.

A technical problem to be solved by the present invention is to provide a directional lead and an identification method for a directional lead. A marking pattern is provided on the directional lead, and a transmission scan image of the directional lead with the marking pattern is acquired, so that medical staffs and researchers can determine and judge the position of each electrode slice according to a two-dimensional transmission scan image photographed in any direction so as to achieve the fixed-point electrical stimulation, thereby avoiding the shooting of the image at multiple angles and the operation of a related apparatus thereof in a surgical process, shortening the orientation time in the surgical process, and reducing the unnecessary radiation to patients in the surgical process.

In the present invention, the marking pattern is arranged on the directional lead and the transmission scan image of the directional lead with the marking pattern is acquired, so that the medical staffs and the researchers determine the direction according to the two-dimensional transmission scan image, whereby the position of each electrode slice is determined and the electric stimulation pulse at the specific point is controlled, thereby shortening the orientation time in the surgical process and the subsequent visit, and reducing the unnecessary radiation to the patients. In the present invention, the electrode slice can be identified by acquiring only one image, which has the advantages of small workload, high speed, high working efficiency and high precision. Since the electrode is a flexible body, considering that the electrode may deviate and rotate a little, the patients may also need to perform positioning, identification, and reexamining, debugging in the later period again, whereby the visit time for the patients and the doctors can be shortened, and the visit cost can be reduced.

## Claims

1. A directional lead, comprising: a connection portion (1), and a plurality of electrode slices (2) circumferentially surrounding the connection portion (1) and arranged in an array, wherein a marking pattern (3) is disposed on a side surface of the connection portion (1), is located on a side of the plurality of electrode slices (2), and at least comprises three distinguishing feature portions, the three distinguishing feature portions are sharp corners or rounded corners;
wherein the directional lead is **characterized in that**, positions of the plurality of electrode slices (2) are capable of being positioned by the distinguishing feature portions of the marking pattern (3) in a two-dimensional transmission scan image photographed in any direction of the directional lead;
wherein the marking pattern (3) comprises a triangular pattern, the triangular pattern is an asymmetric graphic with respect to a first line, and the first line is a line that is parallel to a central axis of the connection portion (1) and located on the side surface of the connection portion (1).

2. The directional lead of claim 1, wherein the marking pattern (3) is composed of a plurality of triangular patterns, and the marking pattern (3) is an asymmetric image with respect to the first line.

3. The directional lead of claim 1, wherein the triangular pattern is an isosceles triangular pattern.

4. The directional lead of claim 3, wherein a bottom edge of the isosceles triangular pattern is parallel to the central axis of the connection portion (1).

5. The directional lead of claim 1, wherein two ends of the marking pattern (3) and a center point of a radial cross-section of the connection portion (1) form an angle of 105° to 135° on the radial cross-section of the connection portion (1).

6. An identification method for a directional lead, comprising
providing a marking pattern (3) in a non-electrode slice region of the directional lead, wherein when acquiring different two-dimensional transmission scan images of the directional lead, imaging positions or shapes of the marking pattern (3) in the different two-dimensional transmission scan images are different; and wherein the directional lead comprises: a connection portion (1), and a plurality of electrode slices (2) circumferentially surrounding the connection portion (1) and arranged in an array, wherein the marking pattern (3) is disposed on a side surface of the connection portion (1), is located on a side of the plurality of electrode slices (2), and at least comprises three distinguishing feature portions, the three distinguishing feature portions are sharp corners or rounded corners;
acquiring, by a transmission optical imaging device, a two-dimensional transmission scan image photographed in any direction of the directional lead; and
positioning, through an imaging of the marking pattern (3) on the two-dimensional transmission scan image, positions of the plurality of electrode slices (2);
wherein the marking pattern (3) comprises a triangular pattern, the triangular pattern is an asymmetric graphic with respect to a first line, and the first line is a line that is parallel to a central axis of the directional lead and located on a side surface of the directional lead.

7. The identification method for the directional lead of claim6, wherein the transmission optical imaging device performs an X-ray scan or an electronic computer tomography scan.

8. The identification method for the directional lead of claim 6, wherein the directional lead comprises a connection portion (1), and a plurality of electrode slices (2) circumferentially surrounding the connection portion and arranged in an array.

## Patentansprüche

1. Richtungsleitung, umfassend: einen Verbindungsabschnitt (1) und eine Vielzahl von Elektrodensegmenten (2), die den Verbindungsabschnitt (1) umfangseitig umgeben und in einem Array angeordnet sind, wobei ein Markierungsmuster (3) auf einer Seitenoberfläche des Verbindungsabschnitts (1) angeordnet ist, auf einer Seite der Vielzahl von Elektrodensegmenten (2) befindlich ist und mindestens drei unterscheidende Merkmalsabschnitte umfasst, wobei die drei unterscheidenden Merkmalsabschnitte scharfe Ecken oder abgerundete Ecken sind,
wobei die Richtungsleitung **dadurch gekennzeichnet ist, dass** Positionen der Vielzahl von Elektrodensegmenten (2) fähig sind, durch die unterscheidenden Merkmalsabschnitte des Markierungsmusters (3) in einem in einer beliebigen Richtung der Richtungsleitung aufgenommenen zweidimensionalen Durchstrahlungsscanbild positioniert zu werden,
wobei das Markierungsmuster (3) ein Dreiecksmuster umfasst, das Dreiecksmuster eine asymmetrische Grafik in Bezug auf eine erste Linie ist und die erste Linie eine Linie ist, die parallel zu einer mittigen Achse des Verbindungsabschnitts (1) verläuft und auf der Seitenoberfläche des Verbindungsabschnitts (1) befindlich ist.

2. Richtungsleitung nach Anspruch 1, wobei das Markierungsmuster (3) aus einer Vielzahl von Dreiecksmustern zusammengesetzt ist und das Markierungsmuster (3) ein asymmetrisches Bild in Bezug auf die erste Linie ist.

3. Richtungsleitung nach Anspruch 1, wobei das Dreiecksmuster ein gleichschenkliges Dreiecksmuster ist.

4. Richtungsleitung nach Anspruch 3, wobei eine unterseitige Kante des gleichschenkligen Dreiecksmusters parallel zu der mittigen Achse des Verbindungsabschnitts (1) verläuft.

5. Richtungsleitung nach Anspruch 1, wobei zwei Enden des Markierungsmusters (3) und ein Mittelpunkt eines radialen Querschnitts des Verbindungsabschnitts (1) einen Winkel von 105° bis 135° auf dem radialen Querschnitt des Verbindungsabschnitts (1) bilden.

6. Identifizierungsverfahren für eine Richtungsleitung, umfassend:
Bereitstellen eines Markierungsmusters (3) in einer Region der Richtungsleitung, die keine Elektrodensegmente aufweist, wobei Abbildungspositionen oder -formen des Markierungsmusters (3) in den unterschiedlichen zweidimensionalen Durchstrahlungsscanbildern unterschiedlich sind, wenn unterschiedliche zweidimensionale Durchstrahlungsscanbilder der Richtungsleitung erfasst werden, und wobei die Richtungsleitung Folgendes umfasst: einen Verbindungsabschnitt (1) und eine Vielzahl von Elektrodensegmenten (2), die den Verbindungsabschnitt (1) umfangseitig umgeben und in einem Array angeordnet sind, wobei das Markierungsmuster (3) auf einer Seitenoberfläche des Verbindungsabschnitts (1) angeordnet ist, auf einer Seite der Vielzahl von Elektrodensegmenten (2) befindlich ist und mindestens drei unterscheidende Merkmalsabschnitte umfasst, wobei die drei unterscheidenden Merkmalsabschnitte scharfe Ecken oder abgerundete Ecken sind;
Erfassen eines in einer beliebigen Richtung der Richtungsleitung aufgenommenen zweidimensionalen Durchstrahlungsscanbilds durch eine optische Durchstrahlungsbildgebungsvorrichtung und
Positionieren von Positionen der Vielzahl von Elektrodensegmenten (2) durch eine Bildgebung des Markierungsmusters (3) auf dem zweidimensionalen Durchstrahlungsscanbild,
wobei das Markierungsmuster (3) ein Dreiecksmuster umfasst, das Dreiecksmuster eine asymmetrische Grafik in Bezug auf eine erste Linie ist und die erste Linie eine Linie ist, die parallel zu einer mittigen Achse der Richtungsleitung verläuft und auf einer Seitenoberfläche der Richtungsleitung befindlich ist.

7. Identifizierungsverfahren für die Richtungsleitung nach Anspruch 6, wobei die optische Durchstrahlungsbildgebungsvorrichtung einen Röntgen-Scan oder einen Computertomografie-Scan durchführt.

8. Identifizierungsverfahren für die Richtungsleitung nach Anspruch 6, wobei die Richtungsleitung einen Verbindungsabschnitt (1) und eine Vielzahl von Elektrodensegmenten (2) umfasst, die den Verbindungsabschnitt umfangseitig umgeben und in einem Array angeordnet sind.

## Revendications

1. Conducteur directionnel, comprenant : une partie de connexion (1), et une pluralité de tranches d'électrode (2) entourant de manière circonférentielle la partie de connexion (1) et disposées selon un réseau, dans lequel un motif de marquage (3) est disposé sur une surface latérale de la partie de connexion (1), est situé sur un côté de la pluralité de tranches d'électrode (2), et comprend au moins trois parties caractéristiques distinctives, les trois parties caractéristiques distinctives sont des coins pointus ou des coins arrondis ;
dans lequel le conducteur directionnel est **caractérisé en ce que**, des positions de la pluralité de tranches d'électrode (2) peuvent être positionnées par les parties caractéristiques distinctives du motif de marquage (3) dans une image de scanner de transmission bidimensionnelle photographiée selon toute direction du conducteur directionnel ;
dans lequel le motif de marquage (3) comprend un motif triangulaire, le motif triangulaire est un graphique asymétrique par rapport à une première ligne, et la première ligne est une ligne qui est parallèle à un axe central de la partie de connexion (1) et située sur la surface latérale de la partie de connexion (1).

2. Conducteur directionnel selon la revendication 1, dans lequel le motif de marquage (3) est composé d'une pluralité de motifs triangulaires, et le motif de marquage (3) est une image asymétrique par rapport à la première ligne.

3. Conducteur directionnel selon la revendication 1, dans lequel le motif triangulaire est un motif triangulaire isocèle.

4. Conducteur directionnel selon la revendication 3, dans lequel un bord inférieur du motif triangulaire isocèle est parallèle à l'axe central de la partie de connexion (1).

5. Conducteur directionnel selon la revendication 1, dans lequel deux extrémités du motif de marquage (3) et un point central d'une section transversale radiale de la partie de connexion (1) forment un angle de 105° à 135° sur la section transversale radiale de la partie de connexion (1).

6. Procédé d'identification pour un conducteur directionnel, comprenant :
la fourniture d'un motif de marquage (3) dans une région de tranche non-électrode du conducteur directionnel, dans lequel, lors de l'acquisition d'images de scanner de transmission bidimensionnelles différentes du conducteur directionnel, des positions ou des formes d'imagerie du motif de marquage (3) dans les différentes images de scanner de transmission bidimensionnelles sont différentes ; et dans lequel le conducteur directionnel comprend : une partie de connexion (1), et une pluralité de tranches d'électrode (2) entourant de manière circonférentielle la partie de connexion (1) et disposées selon un réseau, dans lequel le motif de marquage (3) est disposé sur une surface latérale de la partie de connexion (1), est situé sur un côté de la pluralité de tranches d'électrode (2), et comprend au moins trois parties caractéristiques distinctives, les trois parties caractéristiques distinctives sont des coins pointus ou des coins arrondis ;
l'acquisition, par un dispositif d'imagerie optique de transmission, d'une image de scanner de transmission bidimensionnelle photographiée selon toute direction du conducteur directionnel ; et
le positionnement, au moyen d'une imagerie du motif de marquage (3) sur l'image de scanner de transmission bidimensionnelle, de positions de la pluralité de tranches d'électrode (2) ;
dans lequel le motif de marquage (3) comprend un motif triangulaire, le motif triangulaire est un graphique asymétrique par rapport à une première ligne, et la première ligne est une ligne qui est parallèle à un axe central du conducteur directionnel et située sur une surface latérale du conducteur directionnel.

7. Procédé d'identification pour le conducteur directionnel selon la revendication 6, dans lequel le dispositif d'imagerie optique de transmission effectue une radiographie aux rayons X ou une tomodensitométrie électronique.

8. Procédé d'identification pour le conducteur directionnel selon la revendication 6, dans lequel le conducteur directionnel comprend une partie de connexion (1), et une pluralité de tranches d'électrode (2) entourant de manière circonférentielle la partie de connexion et disposées selon un réseau.
